# EUROPÄISCHE PATENTANMELDUNG

(11) **EP 2 740 418 A1**
(43) Veröffentlichungstag der Anmeldung: **11.06.2014**
(21) Anmeldenummer: 12195521.5
(22) Anmeldetag: 04.12.2012
(51) Int. Cl.: A61B 17/17, A61F 2/46

(54) **Chirurgische Bohrlehre und chirurgischer Werkzeugsatz**

(71) Anmelder: WALDEMAR LINK GmbH & Co. KG, 22339 Hamburg (DE)
(72) Erfinder: Fischer, Hans-Joachim, 22846 Norderstedt (DE); Dmuschewsky, Klaus, 22397 Hamburg (DE)
(74) Vertreter: Raffay & Fleck

(57) **Zusammenfassung**

Es soll eine Bohrlehre (1) für die Führung eines Bohrers beim Einbringen von Befestigungsbohrungen für Befestigungselemente zum Anbringen eines Luxationssicherungselementes an einem Pfannenelement eines künstlichen Hüftgelenks, angegeben werden, die derart beschaffen ist, dass sie auch bei nicht luxiertem Hüftgelenk, also bei in dem Pfannenelement befindlicher Hüftkugel, sicher verwendet werden kann und dabei eine Einstellung des Hüftgelenkes für die Anpassung der Position der Bohrlehre (1) an den aus Sicht des Behandlers bzw. Operateurs richtigen Sitz des Luxationssicherungselementes erlaubt.

Erfindungsgemäß zeichnet sich eine diese Aufgaben lösende Bohrlehre aus durch folgende Elemente:
a) ein Adapterteil (2), welches eine erste Anlagefläche zum Anlegen an den und Abstützen an dem Rand des Pfannenelements und eine zweite Anlagefläche zum Anlegen an eine und Abstützen an einer in dem Pfannenelement aufgenommene Hüftkugel des künstlichen Hüftgelenks aufweist, und
b) ein Werkzeugführungsteil (3) zum Führen des Bohrers, welches kreisabschnittförmig gebogen ist und wenigstens zwei Bohrführungsöffnungen (13) aufweist, wobei das Werkzeugführungsteil (3) an dem Adapterteil (2) festgelegt ist.

## Beschreibung

Die Erfindung betrifft eine Bohrlehre für die Führung eines Bohrers beim Einbringen von Befestigungsbohrungen für Befestigungselemente zum Anbringen eines Luxationssicherungselementes an dem Pfannendach eines künstlichen Hüftgelenkes. Sie betrifft ferner einen chirurgischen Werkzeugsatz zum Setzen und Befestigen eines Luxationssicherungselementes an dem Pfannenelement eines künstlichen Hüftgelenkes.

Es ist bekannt, dass die Ausrenkung (Luxation) eines künstlichen Hüftgelenkes eine der häufigsten Frühkomplikationen nach Versorgung mit einer Hüftendoprothese darstellt. Um derartige Luxationen zu verhindern, werden - typischerweise in der Implantation nachfolgenden Revisionsoperationen - Luxationssicherungselemente, die typischerweise ringabschnittförmig gebildet sind und in dieser typischen Ausgestaltung auch als Luxationssicherungsringe bezeichnet werden, auf den Pfannenrand der künstlichen Hüftpfanne gesetzt und dort fixiert. Entsprechende Luxationssicherungselemente umgreifen die in dem Pfannenelement ruhende Hüftkugel über einen weiteren Abschnitt ihres Umfanges, überragen insbesondere einen Abschnitt, in welchem die Hüftkugel mit ihrem Großkreis liegt und sorgen so für einen sicheren Rückhalt der Hüftkugel in der Gelenkpfanne, verhindern eine Luxation des künstlichen Gelenkes. Da Luxationssicherungselemente einerseits eine Einschränkung der Bewegungsfreiheit des künstliches Gelenkes nach sich ziehen können, andererseits eine Veränderung der auf der Hüftpfannenkomponente der Hüftgelenkendoprothese lastenden Kräfte bewirken (dort bei das Gelenk in axialer Richtung des Oberschenkelhalses wirkenden Zugkräften das Gelenk deutlich höher belasten) werden derartige Luxationssicherungselemente nicht, allenfalls in medizinisch eindeutig indizierten Ausnahmefällen, bereits bei der Primäroperation gesetzt.

In der DE 197 16 051 A1 ist ein Luxationssicherungsring für Pfannenkomponenten von Hüftendoprothesen beschrieben, der aus einem resorbierbaren Material, dort Poly-L-Lactid Acid, besteht und mit aus gleichem Material bestehenden Schrauben fixiert wird. Der Ansatz des dort beschriebenen Luxationssicherungsringes ist, dass er innerhalb einer Rekonvaleszenzzeit (genannt sind dort ca. 6 Wochen) resorbiert und durch körpereigenes Material ersetzt wird. Dieser Luxationssicherungsring soll insbesondere bei der Primäroperation bereits mit gesetzt und an der Gelenkpfannenkomponente fixiert werden.

Insbesondere - aber nicht nur - beim Setzen eines Luxationssicherungselementes in einer Revisionsoperation sind verschiedene Aspekte zu beachten. So ist es einerseits wünschenswert, dass für den gesamten Ablauf der entsprechenden Operation die Hüftkugel in der künstlichen Gelenkpfanne verbleibt, eine Luxation des Hüftgelenkes nicht vorgenommen werden muss. Darüber hinaus gilt es beim Setzen der Befestigungsbohrungen in den Pfannenrand, die für die Festlegung des Luxationssicherungselementes entsprechende Befestigungselemente, typischerweise Befestigungsschrauben, aufnehmen sollen, diese Bohrungen exakt und sicher zu setzen. Diese Bohrungen sollen einerseits nicht zu nah an das Zentrum des Pfannenelementes gelangen, um nicht die darin ruhende Hüftkugel versehentlich anzubohren. Es muss darüber hinaus gewährleistet werden, dass die zwischen der Bohrung und der Führungsfläche für die Hüftkugel verbleibende Wandstärke nicht zu dünn wird, um hier nicht etwa Schwachstellen in dem künstlichen Gelenk zu schaffen. Auf der anderen Seite sollen die Bohrungen jedoch auch nicht zu weit am Außenrand des Pfannenelementes liegen, um auch dort nicht eine zu geringe Wandstärke entstehen zu lassen. Die Bohrungen sollen sicher im Material der Hüftpfanne, welche beispielsweise aus Polyethylen besteht, liegen und nicht etwa in den Knochen hineinragen.

Um entsprechende Bohrungen sicher setzen und anbringen zu können, ist die Verwendung einer entsprechenden Bohrlehre anzustreben. Dabei soll die Bohrlehre insbesondere derart beschaffen sein, dass sie auch bei nicht luxiertem Hüftgelenk, also bei in dem Pfannenelement befindlicher Hüftkugel, sicher verwendet werden kann und dabei eine Einstellung des Hüftgelenkes für die Anpassung der Position der Bohrlehre an den aus Sicht des Behandlers bzw. Operateurs richtigen Sitz des Luxationssicherungselementes erlaubt. Eine solche Bohrlehre zu schaffen ist Aufgabe der vorliegenden Erfindung.

Diese Aufgabe wird erfindungsgemäße gelöst durch eine Bohrlehre mit den Merkmalen des Anspruches 1. Vorteilhafte Weiterbildungen der erfindungsgemäßen Bohrlehre sind in den abhängigen Ansprüchen 2 bis 8 bezeichnet. In einem weiteren Aspekt wird mit der Erfindung zur Lösung der allgemeinen Aufgabe ein chirurgischer Werkzeugsatz mit den Merkmalen des Patentanspruches 9 angegeben, der eine mit der Erfindung gegebene Bohrlehre enthält. Vorteilhafte Weiterbildungen des erfindungsgemäßen Werkzeugsatzes sind in den Ansprüchen 10 bis 14 genannt.

Die erfindungsgemäße Bohrlehre für die Führung eines Bohrers beim Einbringen von Befestigungsbohrungen für Befestigungselemente zum Anbringen eines Luxationssicherungselementes an dem Pfannenelement eines künstlichen Hüftgelenkes enthält wenigstens zwei Teile, nämlich ein Adapterteil und ein Werkzeugführungsteil. Das Adapterteil hat dabei die Aufgabe, die Bohrlehre abhängig vom Modell der beim Patienten implantierten Hüftgelenkendoprothese zur Anlage an derselben anzupassen. Es wird mit anderen Worten spezifisch je nach konkret vorliegender Hüftgelenkendoprothese ausgewählt und in der Bohrlehre verwendet. Es ist dabei mit Anlageflächen so gebildet, dass es sich auf dem Rand des Pfannenelements und zugleich auf der Hüftkugel des künstlichen Hüftgelenks abstützen kann. Dadurch ist es besonders stabil und sicher gestützt und können die Bohrungen geführt durch die Bohrlehre sicher in das Pfannenelement eingebracht werden.

Das Adapterteil hat mit Vorteil drei Abschnitte. Ein erster der drei Abschnitte ist dabei ein teilkreisförmig gebogener, mit einer Oberfläche in einer Ebene verlaufender Randabschnitt, in dem mindestens zwei quer zu der Ebene geführte Bohröffnungen vorgesehen sind. Dieser Randabschnitt dient zum Aufsetzen auf einen Abschnitt eines Randes des Pfannenelementes, nämlich auf denjenigen Abschnitt des Randes des Pfannenelementes, in den die Bohrungen für die Aufnahme der Befestigungselemente zum Sichern des Luxationssicherungselementes an dem Pfannenelement eingebracht werden sollen. Ein zweiter Abschnitt des Adapterteils ist erfindungsgemäß eine sich ausgehend von dem Randabschnitt hin Richtung einer ersten Seite der Ebene quer zu dieser erstreckende, teilkreisförmig parallel zu dem Verlauf des Randabschnittes gebogene Ausrichtleiste. Diese Ausrichtleiste ist typischerweise und bevorzugt in einem relativ zu der Lage der Bohröffnungen in Richtung des konkaven Randes des Randabschnittes, also des in Richtung des Mittelpunktes des Kreisabschnittes gelegenen Abschnittes des Randabschnittes verschobenen Bereich angeordnet. Die Ausrichtleiste wird im Gebrauch des Adapterteiles typischerweise in einen Spalt zwischen dem innen liegenden Rand des Pfannenelementes und der darin geführten Hüftkugel eingesetzt und dient somit der Ausrichtung und Positionierung des Adapterteils. Sie kann aber auch auf einem nach innen geneigt und zur Hüftkugel hin abfallenden Teil des Pfannenrandes aufliegen. Entsprechend ist die Ausrichtleiste in ihrer Stärke ausreichend schmal und an den bestehenden Spalt bzw. die Breite eines nach innen geneigten Abschnittes des Pfannenrandes angepasst zu gestalten. Sie ist dabei mit Vorteil ausgehend von der Ebene hin zu ihrer distalen Kante verjüngt gebildet, kann auf der im Gebrauch der Hüftkugel zugewandten Seite eine sphärenabschnittförmige Kontur aufweisen. Ein dritter Abschnitt des Adapterteils besteht erfindungsgemäß in einem sich ausgehend von dem Randabschnitt über dessen konkave Seite hinaus geführten und ausgehend von der Ebene in Richtung einer zweiten, der ersten Seite der Ebene gegenüberliegenden Seite quer zu der Ebene geführten Kuppelabschnitt. Dieser Kuppelabschnitt weist in einem von dem Randabschnitt entfernten Bereich auf seiner der Ebene des Randabschnittes zugewandten Unterseite einen Auflageabschnitt auf, mit dem er sich bei einer Anordnung des Adapterteils auf dem Rand des Pfannenelementes auf der Hüftkugel abstützt und dadurch zusammen mit einer Unterseite des Randabschnittes und der Ausrichtleiste eine exakte Positionierung des Adapterteils bewirkt.

Das zweite Teil der erfindungsgemäßen Bohrlehre, das Werkzeugführungsteil, dient dem eigentlichen Führen des Bohrers. Dieses Teil ist kreisabschnittförmig gebogen. Wenn das Adapterteil wie oben als vorteilhafte Ausgestaltung dargestellt in den dort beschriebenen drei Abschnitten gebildet ist, folgt das Adapterteil mit Vorteil in seiner Form und Krümmung dem Randabschnitt des Adapterteils, auf dem es dann im Gebrauch aufliegt.

Das Werkzeugführungsteil weist Bohrführungsöffnungen auf, die in ihrer Anordnung und Verteilung den einzubringenden Befestigungsbohrungen entsprechen. Wenn das Adapterteil wie oben als vorteilhafte Ausgestaltung ausgeführt, in Bohröffnungen aufweist, dann entsprechen die Bohrführungsöffnungen in ihrer Anordnung derjenigen der Bohröffnungen in dem Adapterteil. Dabei muss dann nicht notwendigerweise die Anzahl der Bohröffnungen und Bohrführungsöffnungen exakt gleich sein. Es können insbesondere mehr Bohröffnungen als Bohrführungsöffnungen vorhanden sein, so dass das Werkzeugführungsteil eine oder mehrere Bohröffnungen in dem Adapterteil überdeckt und somit zu einer Oberseite des Werkzeugführungsteils hin verschließt.

Das Werkzeugführungsteil ist an dem Adapterteil festgelegt. Ist dass Adapterteil, wie oben ausgeführt, mit Vorteil in den dort genannten Abschnitten ausgebildet, weist insbesondere den Randabschnitt mit den Bohröffnungen auf, so ist das Werkzeugführungsteil an dem Adapterteil so festgelegt, dass es auf dem Randabschnitt des Adapterteils derart positioniert ist, dass seine Bohrführungsöffnungen mit den zugeordneten Bohröffnungen vollständig in Überdeckung liegen. Ein durch die Bohrführungsöffnungen eingeführter Bohrer kann somit durch die Bohröffnungen hindurch in Richtung der Unterseite des Adapterteils, also der dem Werkzeugführungsteil abgewandten Seite des Adapterteils wirken.

Grundsätzlich können das Adapterteil und das Werkzeugführungsteil fest miteinander verbunden sein. Bevorzugt ist jedoch, dass das Werkzeugführungsteil an dem Adapterteil lösbar festlegbar ist, dass diese Teile als getrennte Teile bestehen. Diese Art der Ausgestaltung hat einerseits den Vorteil, dass ein modularer Aufbau möglich ist, dass zum Präparieren der für einen bestimmten operativen Einsatz zu verwendenden Bohrlehre aus unterschiedlichen Adapterteilen und Werkzeugführungsteilen die jeweils passenden gewählt und dann zu der für den spezifischen Gebrauch benötigten Bohrlehre zusammengefügt werden können. Darüber hinaus bietet eine Zerlegbarkeit der Bohrlehre in die Bestandteile Adapterteil und Werkzeugführungsteil eine bessere Möglichkeit der Reinigung und Sterilisierung dieses typischerweise mehrfach zu verwendenden Instrumentes nach Gebrauch im Zuge einer Operation.

Mit Vorteil bestehen bei der erfindungsgemäßen Bohrlehre das Adapterteil und das Werkzeugführungsteil aus unterschiedlichen Materialien. Dabei ist wichtig, dass das Adapterteil aus einem solchen Werkstoff besteht, der bei Anlage an dem Pfannenelement einerseits und der Hüftkugel andererseits das jeweilige Material dort nicht beschädigt. Das Material des Adapterteils ist insoweit insbesondere höchstens so hart wie die Materialien des Pfannenelementes und des Hüftkopfes. Insbesondere wird das Adapterteil dabei aus einem Kunststoff bestehen, z.B. aus Polyethylen (PE). Das Werkzeugführungsteil hingegen hat hinsichtlich der Ansprüche an seinen Werkstoff andere Voraussetzungen zu erfüllen. Hier ist wichtig, dass es im Bereich der Bohrführungsöffnungen einen dorthin durchgeführten Bohrer, der typischerweise aus einem Metall besteht, sicher führen kann, ohne dass es dort zu einem Ausschlagen der Bohrführungsöffnungen kommt, was letztlich zu einer nicht mehr genauen Führung dieses Werkzeuges führen würde. Entsprechend ist das Werkzeugführungsteil aus einem härteren Material zu wählen, z.B. einem gegenüber dem Kunststoff, aus dem das Adapterteil bestehen kann, härteren Kunststoff. Besonders bevorzugt wird das Werkzeugführungsteil jedoch aus einem Metall bestehen.

Die erfindungsgemäße Bohrlehre kann als weiteres Teil zusätzlich zu dem Adapterteil und dem Werkzeugführungsteil ein Griffstück aufweisen. Mit einem solchen Griffstück kann die erfindungsgemäße Bohrlehre besonders einfach positioniert, korrekt ausgerichtet und dann während des Einbringens der Bohrungen gehalten werden. Das Griffstück ist mit besonderem Vorteil an dem Adapterteil festgelegt. Um die Bohrlehre nach einer Anlage an der künstlichen Gelenkpfanne noch für die korrekte Positionierung für die spätere Anbringung des Luxationssicherungselementes an der aus Sicht des Behandlers bzw. Operateurs günstigsten Stelle entlang des Umfanges des Pfannenrandes schieben zu können, hat es sich herausgestellt, dass eine Anbindung des Griffstückes, insbesondere an dem Adapterteil, mit Vorteil gelenkig erfolgen sollte. Hierbei hat sich insbesondere eine kardanische Gelenkverbindung als geeignet erwiesen.

Auch hinsichtlich der Ausgestaltung des Griffstückes ist es besonders günstig, wenn dieses lösbar an den weiteren Komponenten festgelegt ist, insbesondere lösbar an dem Adapterteil. Diese Lösbarkeit ist insbesondere dann von Vorteil, wenn das Griffstück gelenkig an den weiteren Komponenten angeordnet ist. Denn die Gelenkigkeit bedingt Spalträume, die unter der Operation verschmutzen und dann, wenn hier eine Trennung erfolgen kann, einfacher zu reinigen und zu desinfizieren sind für einen nachfolgenden Einsatz der erfindungsgemäßen Bohrlehre. Das Griffstück kann dabei mit Vorteil auf einer in Richtung des Mittelpunktes des Kreisabschnittes gelegenen Stelle auf der der Ebene des Randabschnittes abgewandten Seite des Kuppelabschnittes angebracht sein. Denn ein so in Richtung des Mittelpunktes des Kreisabschnittes des Randabschnittes verlegter Anlagerpunkt des Griffstückes gestattet eine besonders einfache Handhabung, insbesondere bei einer Verschiebung der Bohrlehre in ihrer Position entlang des Umfanges des Pfannenrandes.

Bei der erfindungsgemäßen Bohrlehre sind gemäß einer vorteilhaften Weiterbildung die Bohröffnungen und Bohrführungsöffnungen so ausgestaltet, dass die Öffnungsweite der Bohröffnungen den Durchmesser der Bohrführungsöffnungen übersteigt. Dabei können die Bohröffnungen grundsätzlich kreisförmig sein, sie können aber auch als dem Radius des Randabschnittes folgende Langlöcher gebildet sein, insbesondere um ggf. mit verschiedenen Werkzeugführungsteilen, bei denen Bohrführungsöffnungen unterschiedlich positioniert sind, zusammenwirken zu können. Der Umstand, dass die Durchmesser der Bohrführungsöffnungen geringer sind als die Öffnungsweiten der Bohröffnungen verhindert, dass beim Einbringen der Bohrungen und Führen des Bohrers durch die Bohrführungsöffnungen und Bohröffnungen der Bohrer Material vom Rand des Adapterteils erfasst und dort das Adapterteil beschädigt. Neben einer Beschädigung des Adapterteils würde dies zu einer zusätzlichen Ausbildung von Spänen führen. Die Bildung von Spänen unter der Operation soll minimiert werden, da die Späne nicht im Körper verbleiben dürfen, sondern sorgfältig zu entfernen sind.

In dem wie oben bereits erwähnt zu bevorzugenden Fall, dass Adapterteil und Werkzeugführungsteil lösbar miteinander zu verbinden sind, sind mit Vorteil an diesen Teilen Strukturen ausgebildet für eine lösbare, insbesondere formschlüssige, positionsgetreue Verbindung dieser Elemente miteinander. Diese Strukturen können beispielsweise für eine elastische Schnapp-Verbindung der beiden Teile miteinander sorgen, beispielsweise durch entsprechende Schnapp- bzw. Rastvorsprünge an dem Werkzeugführungsteil und Rücksprünge bzw. Hinterschnitte an dem Adapterteil.

In einem weiteren Aspekt der Erfindung wird ein chirurgischer Werkzeugsatz zum Setzen und Befestigen eines Luxationssicherungselementes an dem Pfannenelement eines künstlichen Hüftgelenkes angegeben, der neben einer wie oben beschriebenen Bohrlehre weiterhin einen Bohrer, Befestigungsmittel zum Fixieren des Luxationssicherungselementes an dem Pfannenelement, eine Haltezange zum Halten der Befestigungsmittel und/oder des Luxationssicherungselementes und ein Befestigungswerkzeug zum Festlegen der Befestigungsmittel aufweist. Mit einem solchen Werkzeugsatz kann im Zuge einer Operation die Stelle, an der das Luxationssicherungselement auf dem Rand des Pfannenelementes der künstlichen Gelenkpfanne fixiert werden soll, durch Setzen der Befestigungsbohrungen vorbereitet werden, und es kann im Anschluss unter Verwendung der Haltezange das Luxationssicherungselement ergriffen und mittels der Befestigungsmittel an dem Pfannenelement fixiert werden. Die Befestigungsmittel können insbesondere Schrauben sein, die in mit einem in der Bohrlehre geführten Bohrer in den Rand des Pfannenelementes eingebrachte Befestigungsbohrungen eingeschraubt werden können.

In dem chirurgischen Werkzeugsatz weist die Haltezange mit Vorteil einen ersten und einen zweiten Zangenschenkel zum Ergreifen und Halten der Befestigungsmittel und/oder des Luxationselementes auf, wobei der erste Zangenschenkel eine an einer distalen Stirnseite mündende Öffnung zum Durchführen des Befestigungswerkzeuges aufweist. Eine solche Ausgestaltung erlaubt es, bei mit der Haltezange gehaltenem Befestigungselement und/oder Luxationssicherungselement das Befestigungswerkzeug zu bedienen und das Befestigungselement in der Befestigungsöffnung zu verankern. Dabei kann die Haltezange an der distalen Stirnseite des ersten Zangenschenkels weiterhin eine Aufnahme zum lösbaren Aufnehmen eines Abschnittes eines Befestigungsmittels aufweisen, wobei die Aufnahme mit der Öffnung derart verbunden ist, dass bei mit einem Abschnitt in der Aufnahme aufgenommenen Befestigungsmittel das Befestigungswerkzeug durch die Öffnung hindurch geführt und an dem Abschnitt des Befestigungsmittels zum Festlegen desselben angreifen kann. Diese Aufnahme kann beispielsweise eine zu einer an die distale Stirnseite angrenzende Seitenfläche hin offenen T-Nut sein, in die ein Schraubenkopf einer Befestigungsschraube als Befestigungselement eingeführt werden kann. Dann ist die Öffnung in dem ersten Zangenschenkel so gebildet und ausgerichtet, dass durch diese ein entsprechendes Schraubwerkzeug, z.B. ein Innensechskant-Schrauber, hindurchgeführt und an einem an dem Schraubenkopf ausgebildete Angriffstruktur, z.B. einem Innensechskant, angreifen kann. So kann mit sicherer axialer Ausrichtung das Befestigungselement, insbesondere eine Befestigungsschraube durch eine entsprechende Durchgangsöffnung des Luxationssicherungselementes hindurch in die Befestigungsbohrung eingeführt und mit dem Befestigungswerkzeug bis zum Erreichen einer Anfangsfestigkeit dort verankert werden. Für den abschließenden Rest der Befestigung kann die Haltezange entfernt und kann mit dem dann frei geführten Befestigungswerkzeug das Befestigungselement endgültig in der Befestigungsbohrung verankert werden.

Diese Haltezange kann losgelöst von dem Werkzeugsatz auch eigenständig Verwendung finden, wird hier insoweit als eigene Erfindung angesehen, die auch ohne die weiteren Bestandteile des erfindungsgemäßen Werkzeugsatzes und insbesondere ohne die erfindungsgemäße Bohrlehre Verwendung finden kann.

Im Falle von Schrauben als Befestigungsmitteln wird bevorzugt, dass diese mit einem selbstschneidend geschliffenen Schraubgewinde versehen sind. Dadurch können diese Schrauben besonders sicher in den Befestigungsbohrungen verankert werden, indem sie sich ein Gegengewinde in dem Material des Pfannenelementes selbst schneiden. Dabei ist es von besonderem Vorteil, wenn der Anschliff der selbstschneidend geschliffenen Schraubgewinde der Schrauben mit einem negativen Winkel ausgeführt ist, so dass beim Einschrauben entstehende Materialspäne in Einschraubrichtung abgeführt werden. Dadurch wird verhindert, dass Späne, die durch die selbstschneidende Schraube entstehen, aus der Befestigungsbohrung herausgeführt werden und so außerhalb des Implantates in den Körper des zu operierenden Patienten gelangen können bzw. dort abgesammelt werden müssen und dann, wenn sie nicht gefunden werden, eine Gesundheitsgefährdung für den Patienten darstellen. Vielmehr werden die Späne, die durch das selbstschneidende Schraubgewinde der Schraube erzeugt werden, in Richtung des Grundes der Befestigungsbohrung geführt und sind dort dann durch die Befestigungsschraube gesichert eingeschlossen.

Ein weiterer Bestandteil des chirurgischen Werkzeugsatzes kann wenigstens ein Befestigungsstift (auch als Pin bezeichnet) sein, dessen Durchmesser dem Durchmesser der mit dem Bohrer ausgebildeten Bohrung entspricht. Mit einem solche Befestigungsstift kann nach dem Setzen der ersten mittels der erfindungsgemäßen Bohrlehre geführten Bohrung diese Bohrlehre in Umfangsrichtung des Randes des Pfannenelementes gesichert werden, indem der Stift durch die Bohrführungsöffnung und die Bohröffnung hindurch bis in die eingebrachte erste Befestigungsbohrung geführt wird. Dann kann die zweite und können ggf. eine dritte oder gar weitere Befestigungsbohrung(en) angebracht werden, ohne dass es zu einer Verschiebung der Bohrlehre entlang des Umfanges des Pfannenrandes kommt.

Weitere Vorteile und Merkmale der Erfindung ergeben sich aus der nachfolgenden Beschreibung eines Ausführungsbeispieles anhand der beigefügten Figuren. Dabei zeigen:
- Fig. 1: eine erfindungsgemäße Bohrlehre in einer ersten Ausgestaltungsvariante und in Explosionsdarstellung;
- Fig. 2: vergrößert einen Ausschnitt der erfindungsgemäßen Bohrlehre mit Adapterteil und Werkzeugführungsteil;
- Fig. 3: vergrößert den Ausschnitt gemäß Fig. 2 in einer Ansicht von schräg unten;
- Fig. 4: das Werkzeugführungsteil der Bohrlehre gemäß Fig. 1;
- Fig. 5: in einer Ansicht von oben Werkzeugführungs- und Adapterteil in einer Zwischenstellung beim Befestigen des Werkzeugführungsteils am Adapterteil;
- Fig. 6: eine Schnittdarstellung durch das Pfannenelement und die Hüftkugel eines künstlichen Hüftgelenkes mit daran angeordnetem künstlichen Gelenkhals mit auf Pfannenelement und Hüftkugel aufgesetzter erfindungsgemäßer Bohrlehre gemäß Fig. 1;
- Fig. 7: eine dreidimensionale Ansicht auf die in Fig. 6 gezeigte Situation mit verschiedenen möglichen Rotationsausrichtungen der Hüftkugel mit daran angeordnetem Gelenkhals;
- Fig. 8: eine Darstellung auf die Anordnung in Fig. 7 von oben mit in einem Abriss angedeuteter, entlang des Umfanges des Pfannenrandes verschobener Position der Bohrlehre,
- Fig. 9: in einer Schnittdarstellung noch einmal und vereinfacht die Situation gemäß Fig. 6;
- Fig. 10: in einer vergleichbaren Schnittdarstellung wie Fig. 9 die Anordnung der Bohrlehre auf dem Rand eines anders gestalteten Pfannenelementes;
- Fig. 11: ein weiteres Ausführungsbeispiel einer erfindungsgemäßen Bohrlehre für die Anordnung auf dem Pfannenrand einer nochmals anders gestalteten Gelenkpfanne eines künstlichen Hüftgelenkes;
- Fig. 12: in einer dreidimensionalen Darstellung eine zu dem erfindungsgemäßen Werkzeugsatz gehörende Haltezange im Zusammenspiel mit einem Befestigungswerkzeug und einem mit der Haltezange zu ergreifenden Befestigungsmittel in Form einer Befestigungsschraube; und
- Fig. 13: in einer teilweise geschnittenen Detailansicht den Endbereich der Zangenschenkel der in Fig. 12 gezeigten Zange zusammen mit dem Befestigungsmittel, einem Abschnitt des Befestigungswerkzeuges und einem mit der Haltezange gehaltenen Luxationssicherungselement.

In den Figuren sind mit keinesfalls maßstabsgerechten, jedoch die wesentlichen Merkmale aufzeigenden Prinzipskizzen Ausführungsbeispiele für eine erfindungsgemäße Bohrlehre sowie als weiteren Bestandteil eines erfindungsgemäßen Werkzeugsatzes eine besonders gestaltete Haltezange dargestellt. Diese Darstellungen und die nachfolgende Beschreibung der Ausführungsbeispiele dienen dabei der Erläuterung und dem tieferen Verständnis der Erfindung, sollen diese indes in keinster Weise beschränken.

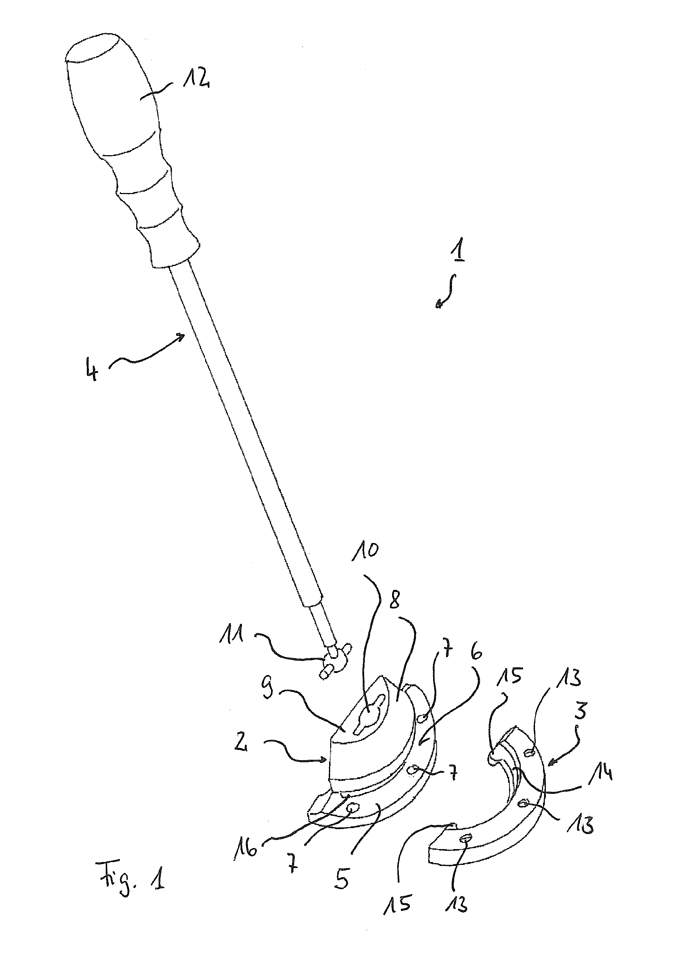

Nachfolgend wird zunächst der Aufbau und die Funktion einer erfindungsgemäßen Bohrlehre in ihrer in den Figuren 1 bis 9 gezeigten ersten Ausführungsvariante erläutert werden.

Die erfindungsgemäße Bohrlehre ist in den Figuren allgemein mit der Bezugsziffer 1 bezeichnet. Sie ist mehrteilig aufgebaut mit unterschiedlichen Funktionsteilen, die in dieser Ausgestaltungsform auch getrennt voneinander bestehen und zum Bilden der Bohrlehre 1 lösbar zusammengefügt werden.

Die einzelnen Teile der Bohrlehre 1 sind ein Adapterteil 2, ein Werkzeugführungsteil 3 und ein Griffteil 4.

Das Adapterteil 2 weist einen in einem Kreisbogen geführten Randabschnitt 5 mit einer planen und ebenen Oberfläche 6 auf, wobei in dem Randabschnitt 5 und quer zu der Oberfläche 6 insgesamt drei Bohröffnungen 7 geführt sind. Ausgehend von der Ebene der Oberfläche 6 in einer Richtung, in Fig. 1 nach oben, erstreckt sich ein Kuppelabschnitt 8. Dieser Kuppelabschnitt 8 hat eine im Wesentlichen parallel zu der Ebene der Oberfläche 6 geführte, gerade Abschlussfläche 9 mit einer darin ausgebildeten Kardanaufnahme 10. Diese Kardanaufnahme 10 dient zur Aufnahme einer Kardan-Gelenkkugel 11 an einem distalen Ende des Griffstückes 4. An dem dem distalen Ende mit der Kardan-Gelenkkugel 11 gegenüberliegenden proximalen Ende weist das entlang einer Längsachse langgestreckte Griffstück 4 einen Handgriff 12 auf.

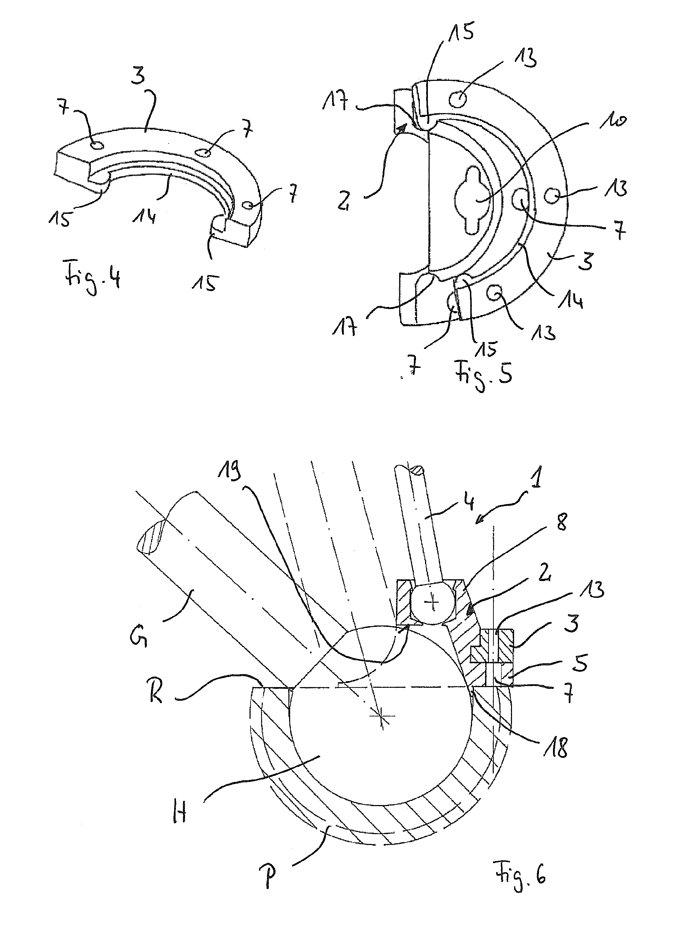

Das Werkzeugführungsteil 3 ist ebenfalls gekrümmt über einen Kreisbogenabschnitt geführt, wobei der Krümmungsradius dieses Kreisbogenabschnittes dem Krümmungsradius des Randabschnittes 5 des Adapterteils 2 entspricht. In das Werkzeugführungsteil 3 sind insgesamt drei Bohrführungsöffnungen 13 eingebracht. Diese Bohrführungsöffnungen 13 sind in ihrer Anordnung und Verteilung so über das Werkzeugführungsteil 3 verteilt, dass sie bei an dem Adapterteil 2 festgelegten Werkzeugführungsteil 3 mit den Bohröffnungen 7 fluchten, von diesen vollständig überdeckt werden. Dabei sind die Durchmesser der Bohröffnungen 7 größer als die Durchmesser der Bohrführungsöffnungen 13 (vgl. insbesondere Fig. 6).

Das Werkzeugführungsteil 3 weist ferner einen nach innen in Richtung des Krümmungsmittelpunktes gerichteten Steg 14 mit an den Randbereichen des Steges 14 vorspringende Rastnasen 15 auf. Im zusammengefügten Zustand sitzt der Steg 14 des Werkzeugführungsteils 3 in einem Hinterschnitt 16 des Adapterteils 2, und die Rastnasen 15 hintergreifen korrespondierende Ausnehmungen 17 im Material des Adapterteils 2 (vgl. Fig. 5).

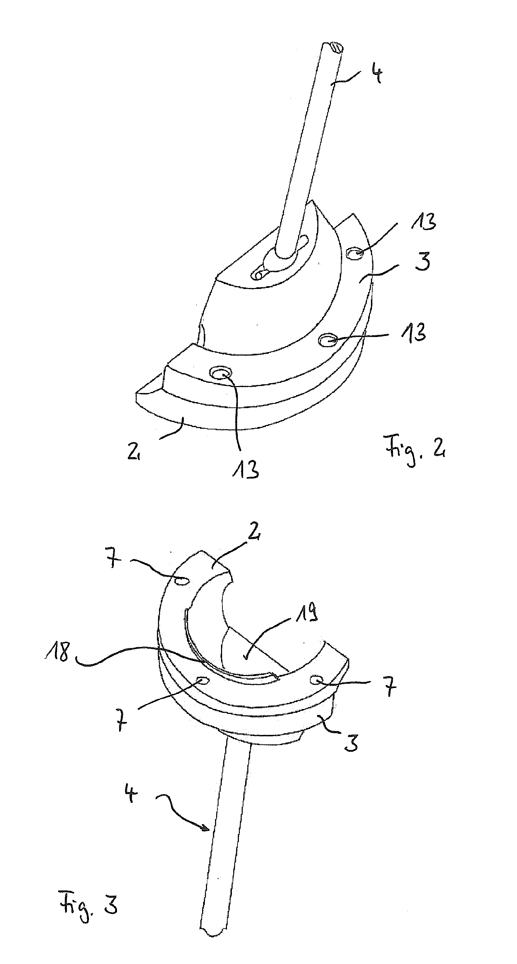

Bei dem gezeigten Ausführungsbeispiel ist ferner auf der in Fig. 3 gezeigten, sich auf die dem Kuppelabschnitt 8 gegenüberliegende Seite der Ebene der Oberfläche 6 erstreckende Unterseite weisend eine gekrümmt verlaufende, auf einer in Richtung des Krümmungszentrums weisenden Innenseite des Adapterteils 2 gelegene Ausrichtleiste 18 zu erkennen (vgl. Fig. 3). In Fig. 3 ist ebenso eine der Abschlussfläche 9 des Kuppelabschnittes 8 gegenüberliegende Anlagefläche 19 zu erkennen, die im Wesentlichen parallel verläuft zu der Oberfläche 6 und der Anlage an einer Hüftkugel eines künstlichen Hüftgelenks dient und dort einen zusätzlichen Auflagepunkt für das Adapterteil 2 und damit die Bohrlehre 1, wenn das Adapterteil 2 mit seinem Randabschnitt 5 auf dem Rand eines Pfannenelementes des künstlichen Hüftgelenks aufgesetzt ist.

In den Figuren 6 bis 9 ist nun die Funktionsweise der erfindungsgemäßen Bohrlehre veranschaulicht, sie wird nachstehend erläutert.

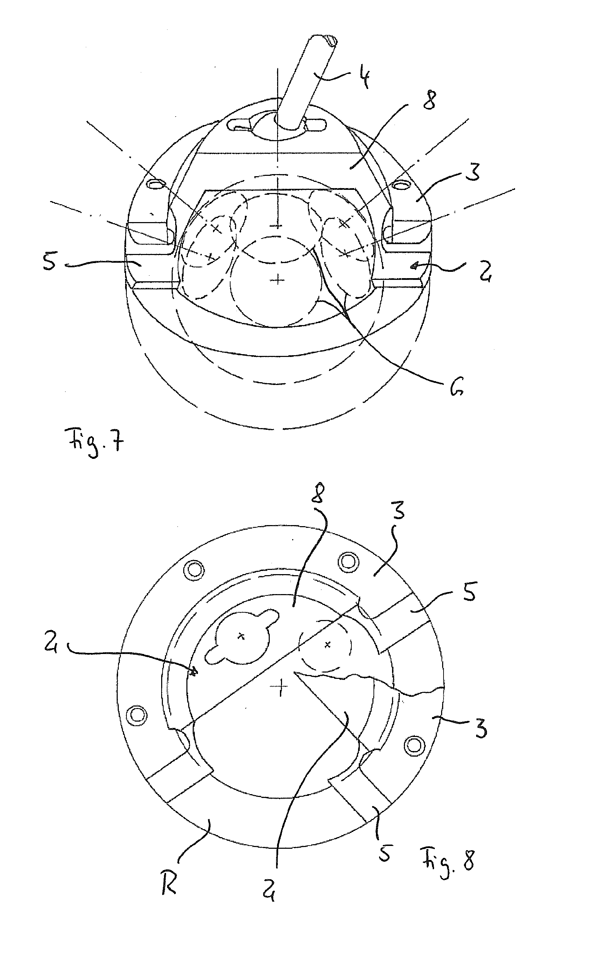

In Fig. 6 ist zunächst ein Pfannenelement P mit einer darin aufgenommenen Hüftkugel H und dem an der Hüftkugel H angeformten Gelenkhals G zu erkennen. Alle zuletzt genannten Elemente, d.h. Pfannenelement P, Hüftkugel H und Gelenkhals G sind Bestandteile einer Hüftgelenkendoprothese, also eines künstlichen Hüftgelenkes. In Fig. 6 ist nun zu erkennen, wie die erfindungsgemäße Bohrlehre 1 an ein bestehendes und zusammengefügtes Hüftgelenk angesetzt, insbesondere mit dem Randabschnitt 5 des Adapterteils 2 auf den Rand R des Pfannenelementes P aufgesetzt wird. In dieser Position stützt sich der Randabschnitt 5 auf dem Rand R des Pfannenelementes P ab. Weiterhin ist die Anlagefläche 19 des Kuppelabschnittes 8 des Adapterteils 2 auf der Hüftkugel H abgestützt. Eine weitere Abstützung erfährt das Adapterteil 2 im Bereich der Ausrichtleiste 18, die in einen Spalt zwischen dem Pfannenelement P und der Hüftkugel H eingreift, der im Bereich des Randes R des Pfannenelementes P besteht. In dieser Situation ist das Adapterteil 2 sicher und zuverlässig abgestützt, kann nicht verkippen oder radial entlang des Randes R des Pfannenelementes P verschoben werden. Mit Hilfe des Griffteils 4 kann es in dieser Position gehalten werden. Bereits in Fig. 6 ist durch die gestrichelt verschobene Lage des Gelenkhalses G dargestellt, dass mit dem aufgesetzten Adapterteil die Hüftkugel H in dem Pfannenelement P verdreht und damit das künstliche Gelenk je nach Bedarf unterschiedlich ausgerichtet werden kann. Dies ist auch noch einmal in Fig. 7 mit insgesamt sechs angedeuteten unterschiedlichen Stellungen des Gelenkhalses G veranschaulicht. Dadurch kann die Positionierung der Bohrlehre 1 und damit die Auswahl des Bereichs des Pfannenelements P, an dem ein Luxationssicherungselement angeordnet werden soll, noch besser erfolgen, indem das Hüftgelenk entsprechend so ausgerichtet wird, dass dieser Bereich gut zugänglich ist.

Die Aufsicht nach Fig. 8 lässt einerseits erkennen, dass der Randabschnitt 5 des Adapterteils 2 in etwa halbkreisförmig ausgebildet ist, wohingegen der Kuppelabschnitt 8 weiter zurückspringt, somit dem Gelenkhals G einen weitergehenden Bewegungsspielraum einräumt. Auch ist zu erkennen, dass das Werkzeugführungsteil 3 über einen geringeren Winkelbereich sich entlang des Umfanges des Randabschnittes 5 des Adapterteils 2 erstreckt. Angedeutet ist hier auch mit einer abgerissen dargestellten, weiter nach rechts unten in der Figur verschobenen Position des Adapterteils 2, wie die Bohrlehre 1 entlang des Umfanges auf dem Rand R des Pfannenelementes verschoben werden kann zur Positionierung der Bohrlehre in dem Bereich, in dem der Behandler bzw. Operateur das Luxationssicherungselement zu setzen gedenkt. Dabei wird die Bohrlehre 1, genauer deren Adapterteil 2, durch die Ausrichtleiste 18 radial geführt.

In dem gezeigten Ausführungsbeispiel besteht das Adapterteil 2 aus einem Kunststoff, insbesondere Polyethylen, wohingegen das Werkzeugführungsteil 3 und das Griffteil 4 aus einem Metall, insbesondere Edelstahl, gebildet sind. Für die Verwendung wird in einem ersten Schritt die Bohrlehre 1 durch Zusammenfügen der Elemente Adapterteil 2, Werkzeugführungsteil 3 und Griffteil 4 aufgebaut. Dazu wird, wie in Fig. 5 gezeigt, das Werkzeugführungsteil 3 über das Adapterteil 2 geschoben, so dass dieses elastisch und formschlüssig durch Eingriff der Rastnasen 15 in die Ausnehmungen 17 sicher an diesem Element sitzt. Der Steg 14 verhindert durch seinen Eingriff in den Hinterschnitt 16 ein Abheben des Werkzeugführungsteils 3 von dem Adapterteil 2 in einer Richtung quer zur Erstreckung der Oberfläche 6. Das Griffteil 4 wird durch Eindrücken der Kardan-Gelenkkugel 11 in die Kardanaufnahme 10, deren Öffnungsweite geringfügig kleiner bemessen ist als die Außenabmessungen der Kardan-Gelenkkugel 11, in diese Aufnahme 10 eingebracht, wobei das Material des Adapterteils 2 im Bereich der Kardanaufnahme 10 elastisch ausweicht und die gelenkige Anbindung des Griffteils 4 durch eine Rast- bzw. Schnappverbindung herstellt.

Die so erhaltene Bohrlehre wird im Falle einer Revisionsoperation auf den freigelegten Rand R das Pfannenelementes P aufgesetzt und entlang des Umfanges solange verschoben, bis sie an der Position sitzt, an der der Operateur das Luxationssicherungselement anzubringen beabsichtigt. Durch die wie in Fig. 6 gezeigte und vorstehend beschriebene sichere Abstützung der Bohrlehre 1 mit ihrem Adapterelement 2 sind die Bohrführungsöffnungen 13 sicher und exakt ausgerichtet, es kann nun durch diese hindurch ein Bohrer eingeführt und es können die Befestigungsbohrungen in das Material des Pfannenelementes P von dessen Rand R her eingebracht werden. Für das Einhalten der maximalen Bohrtiefe kann der Bohrer mit einem Vorsprung versehen sein, mit dem dieser am Rand der Bohrführungsöffnung 13 auf der freiliegenden Oberseite des Werkzeugführungsteils 3 anschlägt, mithin keine tiefer gehende Bohrung mehr erlaubt.

Nach dem Einbringen der erste Befestigungsbohrung kann die erfindungsgemäße Bohrlehre 1 durch Hindurchführen eines Befestigungsstiftes (eines sogenannten Pins) durch die Bohrführungsöffnung 13 in die eingebrachte Befestigungsbohrung in Umfangsrichtung gesichert werden, um für das Einbringen der weiteren Befestigungsbohrungen ein etwaiges Verschieben entlang des Umfanges zu verhindern, mithin sicherzustellen, dass auch die weiteren Befestigungsbohrungen exakt positioniert sind.

Auf gleiche Weise kann, falls dies erforderlich sein sollte, selbstverständlich die Bohrlehre 1 auch bei einer Primäroperation eingesetzt werden, falls bereits dort ein Luxationssicherungselement mit implantiert werden soll.

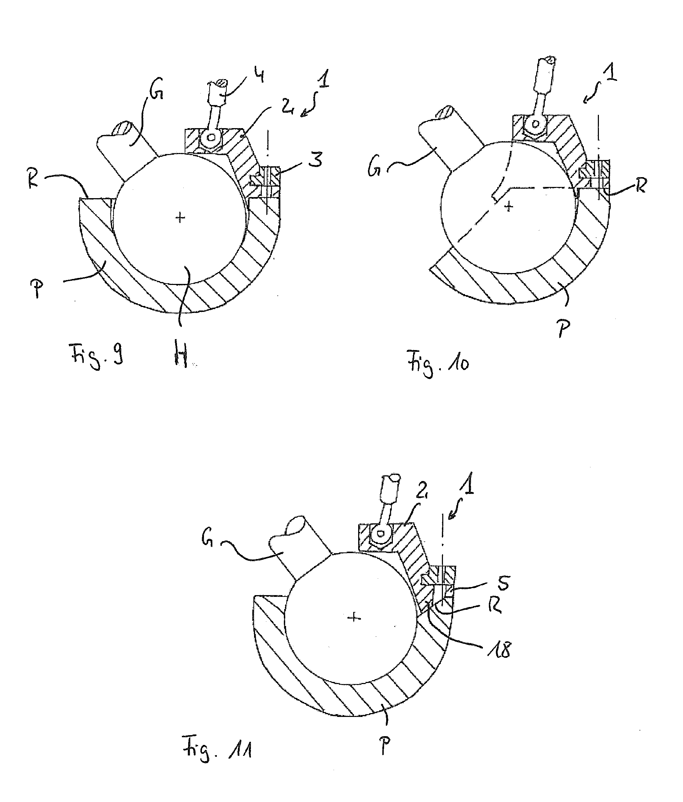

In Fig. 9 ist noch einmal in kleinerer Ansicht die vorstehend beschriebene Bohrlehre 1 aufgesetzt auf dem auch in Fig. 6 gezeigten Pfannenelement P eines künstlichen Hüftgelenks dargestellt. In den Figuren 10 und 11 sind abweichende Ausgestaltungsformen dargestellt, die auf anders geformten Pfannenelementen zum Einsatz kommen können. Insbesondere bei der Bohrlehre 1 in Fig. 11 ist das Adapterteil 2 im Bereich des Randabschnittes 5 anders geformt. Dort ist eine verbreiterte Ausrichtleiste 18 vorgesehen, die an einem schräg nach innen verlaufenden Abschnitt des Pfannenrandes R anliegt und dort eine Ausrichtung und Abstützung des Adapterteils 2 bewirkt.

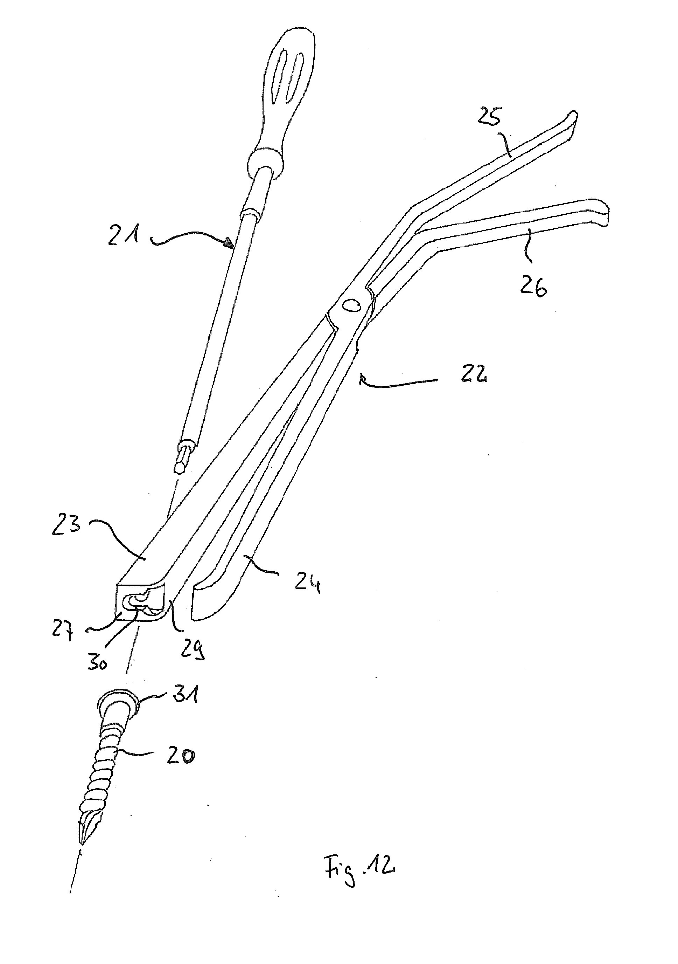

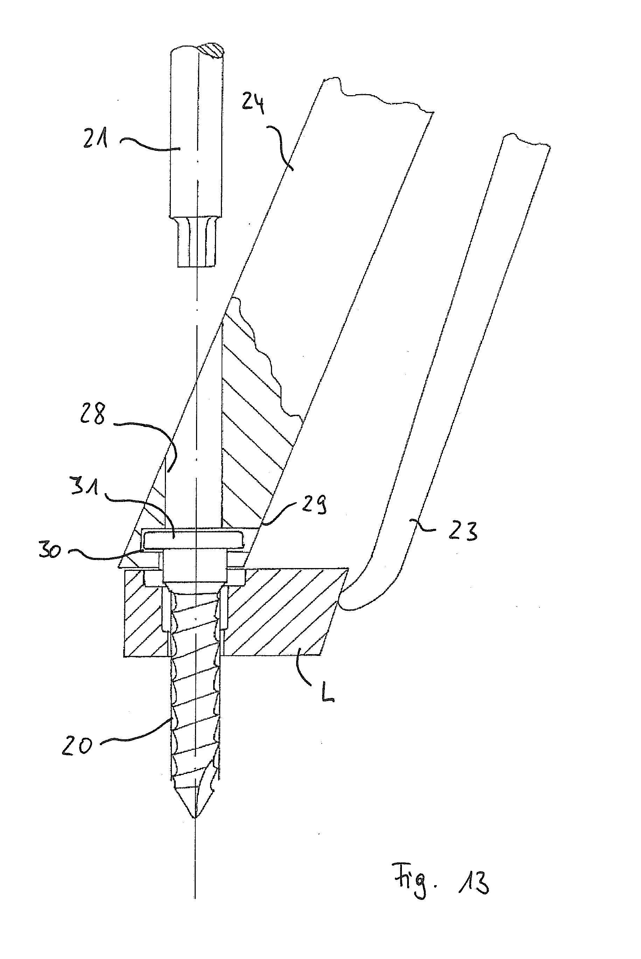

In den Figuren 12 und 13 sind weitere Bestandteile eines eine Bohrlehre 1 wie vorstehend beschrieben enthaltenen chirurgischen Werkzeugsatzes gezeigt. Dazu gehören ein Befestigungselement in Form einer mit einem selbstschneidendem Gewinde ausgeführten Befestigungsschraube 20, ein Befestigungswerkzeug in Form eines Schraubendrehers 21 mit Sechskantanschluss sowie eine Haltezange 22 zum Halten der Befestigungsschraube 20 und/oder eines Luxationssicherungselementes, wie des in Fig. 13 im Schnitt dargestellten Luxationssicherungsringes L.

Die Haltezange 22 weist zwei Zangenschenkel 23 und 24 auf, die durch Betätigen der Griffenden 25 und 26 aufeinander zu bzw. voneinander weg bewegt werden können. Der erste Zangenschenkel 23 ist dabei von einer an dessen Stirnseite 27 mündenden durchgehenden Öffnung 28 durchdrungen, durch die hindurch der Schraubendreher 21 geführt werden kann. Weiterhin ist an der Stirnseite 27 eine zu der dem zweiten Zangenschenkel 24 zugewandten Innenseite 29 hin offene T-Nut 30 eingebracht. In diese T-Nut 30 kann von der Innenseite 29 her der Kopf 31 der Befestigungsschraube 20 eingebracht werden, so dass dieser Kopf 31 in einer Position ruht, in der die Befestigungsschraube 20 in ihrer axialen Richtung mit der axialen Richtung der Öffnung 28 fluchtet und der Werkzeugangriffsabschnitt, hier ein Innensechskant, an dem Schraubenkopf 31 durch die Öffnung 28 für den Sechskantanschluss des Schraubendrehers 21 (oder einen anders geformten Werkzeuganschluss an dem Schraubendreher 21) erreicht und betätigt werden kann. Dabei ist der zweite Zangenschenkel 24 der Haltezange 22 so gestaltet, dass dieser entweder an einem mit der Schraube 20 gemeinsam gehaltenen Luxationssicherungsring L angegriffen werden kann oder aber, wenn dieser Luxationssicherungsring L nicht mit transportiert werden soll, z.B. zum Zuführen weiterer Befestigungsschrauben 20 zu einem bereits auf dem Pfannenrand abgelegten und mit einer Schraube 20 vorgesicherten Luxationssicherungsring L, der Zangenschenkel 23 unmittelbar an einer mit ihrem Kopf 31 in die T-Nut 30 eingeführten Schraube 20 angelegt werden kann, um diese Schraube 20 während des Zuführens und eines ersten Befestigungsschrittes zu halten. Bevor die Befestigungsschraube 20 vollständig in die Befestigungsbohrung eingeschraubt ist, wird die T-Nut über den Kopf 31 der Schraube 20 geführt und der Zangenschenkel 24 von dem Schraubenkopf 31 gelöst. Sodann kann die Befestigungsschraube 20 final festgezogen und der Luxationssicherungsring L so auf dem Pfannenrand P festgelegt werden.

Die Befestigungsschraube 20 ist mit einem selbstschneidenden Gewinde versehen mit einen Anschliff unter negativem Winkel, so dass sie die entstehenden Späne in den Grund der Bohrung abgeführt werden.

### Bezugszeichenliste

- 1: Bohrlehre
- 2: Adapterteil
- 3: Werkzeugführungsteil
- 4: Griffteil
- 5: Randabschnitt
- 6: Oberfläche
- 7: Bohröffnung
- 8: Kuppelabschnitt
- 9: Abschlussfläche
- 10: Kardanaufnahme
- 11: Kardan-Gelenkkugel
- 12: Handgriff
- 13: Bohrführungsöffnung
- 14: Steg
- 15: Rastnase
- 16: Hinterschnitt
- 17: Ausnehmung
- 18: Ausrichtleiste
- 19: Anlagefläche
- 20: Befestigungsschraube
- 21: Schraubendreher
- 22: Haltezange
- 23: Zangenschenkel
- 24: Zangenschenkel
- 25: Griffende
- 26: Griffende
- 27: Stirnseite
- 28: Öffnung
- 29: Innenseite
- 30: T-Nut
- 31: Kopf

- G: Gelenkhals
- H: Hüftkugel
- L: Luxationssicherungsring
- P: Pfannenelement
- R: Rand

## Patentansprüche

1. Bohrlehre für die Führung eines Bohrers beim Einbringen von Befestigungsbohrungen für Befestigungselemente (20) zum Anbringen eines Luxationssicherungselementes (L) an einem Pfannenelement (P) eines künstlichen Hüftgelenks, **gekennzeichnet durch**
a) ein Adapterteil (2), welches eine erste Anlagefläche zum Anlegen an den und Abstützen an dem Rand (R) des Pfannenelements (P) und eine zweite Anlagefläche (19) zum Anlegen an eine und Abstützen an einer in dem Pfannenelement (P) aufgenommene Hüftkugel (H) des künstlichen Hüftgelenks aufweist, und
b) ein Werkzeugführungsteil (3) zum Führen des Bohrers, welches kreisabschnittförmig gebogen ist und wenigstens zwei Bohrführungsöffnungen (13) aufweist, wobei das Werkzeugführungsteil (3) an dem Adapterteil (2) festgelegt ist.

2. Bohrlehre nach Anspruch 1, **dadurch gekennzeichnet, dass** das Adapterteil (2) folgende Abschnitte aufweist:
i. einen teilkreisförmig gebogenen, mit einer Oberfläche in einer Ebene verlaufenden Randabschnitt (5) zum Aufsetzen auf einen Abschnitt des Rands (R) des Pfannenelements (P), wobei in dem Randabschnitt (5) mindestens zwei quer zu der Ebene geführte Bohröffnungen (7) vorgesehen sind,
ii. eine sich von dem Randabschnitt (5) ausgehend in Richtung einer ersten Seite der Ebene quer zu dieser erstreckenden, teilkreisförmig parallel zu dem Verlauf des Randabschnittes (5) gebogenen Ausrichtleiste (18) und
iii. einen sich ausgehend von dem Randabschnitt (5) über dessen konkave Seite hinaus geführten und ausgehend von der Ebene in Richtung einer zweiten, der ersten Seite der Ebene gegenüberliegenden Seite quer dazu geführten Kuppelabschnitt (8), der in einem von dem Randabschnitt (5) entfernten Bereich auf einer der Ebene des Randabschnitts (5) zugewandten Unterseite einen Auflageabschnitt (19) zum Abstützen auf der in dem Pfannenelement (P) gelagerten Hüftkugel (H) aufweist,
und dass das Werkzeugführungsteil (3) an dem Adapterteil (2) so festgelegt ist, dass es auf dem Randabschnitt (5) des Adapterteils (2) derart positioniert ist, dass seine Bohrführungsöffnungen (13) mit den zugeordneten Bohröffnungen (7) vollständig in Überdeckung liegen.

3. Bohrlehre nach Anspruch 2, **dadurch gekennzeichnet, dass** die Öffnungsweite der Bohröffnungen (7) den Durchmesser der Bohrführungsöffnungen (13) übersteigt.

4. Bohrlehre nach einem der vorhergehenden Ansprüche, **dadurch gekennzeichnet, dass** das Adapterteil (2) aus einem Kunststoff, das Werkzeugführungsteil (3) aus einem Metall besteht.

5. Bohrlehre nach einem der vorhergehenden Ansprüche, **dadurch gekennzeichnet, dass** sie weiterhin an dem Adapterteil (2) ein Griffstück (4) aufweist.

6. Bohrlehre nach Anspruch 5, **dadurch gekennzeichnet, dass** das Griffstück (4) an dem Adapterteil (2) gelenkig festgelegt ist, insbesondere mit einer kardanischen Gelenkverbindung.

7. Bohrlehre nach einem der Ansprüche 5 oder 6, **dadurch gekennzeichnet, dass** das Griffstück (4) lösbar an dem Adapterteil (2) festgelegt ist.

8. Bohrlehre nach einem der vorhergehenden Ansprüche, **dadurch gekennzeichnet, dass** an dem Adapterteil (2) und dem Werkzeugführungsteil (3) Strukturen (15, 17) ausgebildet sind für eine lösbare, insbesondere formschlüssige, positionsgetreue Verbindung dieser Elemente (2, 3) miteinander.

9. Chirurgischer Werkzeugsatz zum Setzen und Befestigen eines Luxationssicherungselements (L) an dem Pfannenelement (P) eines künstlichen Hüftgelenks umfassend:
a) eine Bohrlehre (1) nach einem der vorhergehenden Ansprüche,
b) einen Bohrer,
c) Befestigungsmittel (20) zum Fixieren des Luxationssicherungselements (L) an dem Pfannenelement (P),
d) eine Haltezange (22) zum Halten der Befestigungsmittel (20) und/oder des Luxationssicherungselements (L),
e) ein Befestigungswerkzeug (21) zum Festlegen der Befestigungsmittel (20).

10. Chirurgischer Werkzeugsatz nach Anspruch 9, **dadurch gekennzeichnet, dass** die Haltezange (22) einen ersten (23) und einen zweiten (24) Zangenschenkel zum Ergreifen und Halten der Befestigungsmittel (20) und/oder des Luxationssicherungselementes (L) aufweist, wobei der erste Zangenschenkel (23) eine an einer distalen Stirnseite (27) mündende Öffnung (28) zum Durchführen des Befestigungswerkzeugs (21) aufweist.

11. Chirurgischer Werkzeugsatz nach Anspruch 10, **dadurch gekennzeichnet, dass** die Haltezange (22) an der distalen Stirnseite (27) des ersten Zangenschenkels (23) eine Aufnahme (30) zum lösbaren Aufnehmen eines Abschnitts (31) eines Befestigungsmittels (20) aufweist, wobei die Aufnahme (30) mit der Öffnung (28) derart verbunden ist, dass bei mit einem Abschnitt (31) in der Aufnahme (30) aufgenommenem Befestigungsmittel (20) das Befestigungswerkzeug (21) durch die Öffnung (28) hindurch geführt und an dem Abschnitt (31) des Befestigungsmittels (20) zum Festlegen desselben angreifen kann.

12. Chirurgischer Werkzeugsatz nach einem der Ansprüche 9 bis 11, **dadurch gekennzeichnet, dass** die Befestigungsmittel (20) Schrauben mit einem selbstschneidend geschliffenen Schraubgewinde sind.

13. Chirurgischer Werkzeugsatz nach Anspruch 12, **dadurch gekennzeichnet, dass** der Anschliff der selbstschneidend geschliffenen Schraubgewinde der Schrauben mit einem negativen Winkel ausgeführt ist, um so beim Einschrauben entstehende Materialspäne in Einschraubrichtung abzuführen.

14. Chirurgischer Werkzeugsatz nach einem der Ansprüche 9 bis 13, **dadurch gekennzeichnet, dass** er weiterhin wenigstens einen Befestigungsstift aufweist, dessen Durchmesser dem Durchmesser der mit dem Bohrer ausgebildeten Bohrung entspricht.
